(19) Europäisches Patentamt
European Patent Office
Office européen des brevets

(11) EP 4 194 511 A1

(12) **EUROPEAN PATENT APPLICATION**
published in accordance with Art. 153(4) EPC

(43) Date of publication:
**14.06.2023 Bulletin 2023/24**

(21) Application number: **21854641.4**

(22) Date of filing: **05.08.2021**

(51) International Patent Classification (IPC):
***C08L 77/04*** *(2006.01)* ***C08G 63/08*** *(2006.01)*
***C08G 63/91*** *(2006.01)* ***C08J 3/28*** *(2006.01)*
***C08J 3/24*** *(2006.01)* ***A61L 24/00*** *(2006.01)*
***A61L 24/06*** *(2006.01)* ***A61L 31/14*** *(2006.01)*
***A61L 31/04*** *(2006.01)*

(52) Cooperative Patent Classification (CPC):
**A61L 24/00; A61L 24/06; A61L 31/04; A61L 31/14; C08G 63/08; C08G 63/91; C08J 3/24; C08J 3/28; C08L 77/04**

(86) International application number:
**PCT/KR2021/010330**

(87) International publication number:
**WO 2022/031073 (10.02.2022 Gazette 2022/06)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **06.08.2020 KR 20200098415**

(71) Applicant: **Samyang Holdings Corporation**
**Seoul 03129 (KR)**

(72) Inventors:
- **CHANG, Ji Hyun**
  **Seongnam-si Gyeonggi-do 13494 (KR)**
- **KIM, Hyung Hwan**
  **Seongnam-si Gyeonggi-do 13504 (KR)**
- **YOON, Hye Sung**
  **Yongin-si Gyeonggi-do 16827 (KR)**

(74) Representative: **Wohlfahrt, Jan Günther et al**
**Gleiss Große Schrell und Partner mbB**
**Patentanwälte Rechtsanwälte**
**Leitzstraße 45**
**70469 Stuttgart (DE)**

# (54) COMPOSITION FOR HYDROGEL FORMATION, HYDROGEL FORMED BY PHOTO-CROSSLINKING SAME, AND METHOD FOR PREPARING HYDROGEL

(57) The present invention relates to a composition for hydrogel formation, a hydrogel formed by photo-crosslinking same, and a method for preparing the hydrogel and, more specifically, to a composition which comprises a photo-initiator and a polyglutamic acid polymer having a photo-crosslinkable functional group introduced thereto and is capable of forming a hydrogel in real time through photo-crosslinking, to a hydrogel which is formed by photo-crosslinking the composition and can be utilized as a tissue adhesive, a surgical sealant, anti-adhesive agent, or the like due to excellent biocompatibility, superior adhesive strength in wet environments, flexibility, and ease of use, and to a method for preparing the hydrogel.

【FIGURE 2】

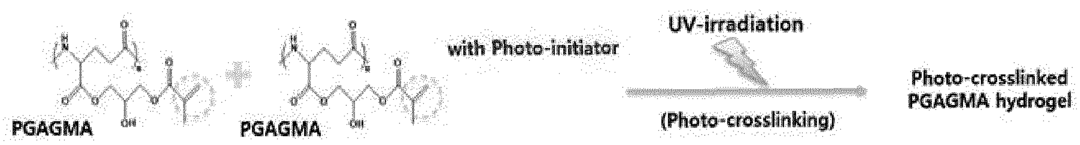


EP 4 194 511 A1

## Description

### TECHNICAL FIELD

The present invention relates to a composition for forming hydrogel, a hydrogel formed by photo-crosslinking the same, and a method for preparing the hydrogel, and more specifically, a composition which comprises a polyglutamic acid polymer having a photo-crosslinkable functional group introduced therein and a photo-initiator, and is capable of forming a hydrogel in situ through photo-crosslinking, a hydrogel which is formed by photo-crosslinking the composition, and has excellent biocompatibility and adhesive strength in wet environment, flexibility, and ease of use so that it can be utilized as tissue adhesive, surgical sealant, adhesion barrier, etc., and a method for preparing the hydrogel.

### BACKGROUND ART

Products such as tissue adhesives, surgical sealants and agents for preventing adhesion between tissues are applied to tissues such as dura mater, blood vessels, cornea, lung, intestine, etc. during surgery to help patients recover after surgery through roles of sealing and wound healing, or applied to surgical areas where adhesion between tissues can be formed, and play a role of preventing formation of the adhesion between tissues.

The currently approved tissue adhesives and sealants include cyanoacrylate-based adhesive (Dermabond by Ethicon), fibrin gel utilizing blood coagulation mechanism (Tisseel by Baxter), PEG-based synthetic sealant (Duraseal by Integra lifesciences), etc.

However, in spite of strong adhesion strength thereof, cyanoacrylate-based adhesive has a problem in safety due to cytotoxicity. Fibrin gel has a risk of infection due to the use of ingredient derived from bovine or human source, and it also has low viscosity and mechanical strength, by which in some cases it can neither be applied to the desired area nor perform the function of sealing properly. PEG-based synthetic sealant has good physical properties as a sealant, but has a problem in that it lacks adhesive strength in wet environment and falls off from organs having repetitive movements without being attached thereto. In addition, since the above products require separate procedures of dissolution and assembly before use, usability is low.

Accordingly, there is a demand for the development of new materials that can be suitably used as tissue adhesive, surgical sealant, adhesion barrier, etc., without the above-mentioned disadvantages of the prior art.

### CONTENTS OF THE INVENTION

### PROBLEMS TO BE SOLVED

The purpose of the present invention is to provide a composition capable of forming a hydrogel which has excellent biocompatibility, safety with low cytotoxicity, excellent adhesion to tissues in wet environment, flexibility, and ease of use in the medical field, and thus can be utilized as tissue adhesive, surgical sealant, adhesion barrier, etc., and a hydrogel formed by photo-crosslinking the composition.

### TECHNICAL MEANS

To achieve the above purpose, the present invention provides a composition for forming hydrogel, comprising: a poly($\gamma$-glutamic acid) (PGA) having a photo-crosslinkable functional group introduced therein; and a photo-initiator.

In an embodiment, the composition for forming hydrogel may be a composition for forming hydrogel, comprising: a first component comprising a poly($\gamma$-glutamic acid) (PGA) having a photo-crosslinkable functional group introduced therein; and a second component comprising a photo-initiator.

The present invention also provides a hydrogel formed by photo-crosslinking the above composition for forming hydrogel of the present invention.

The present invention also provides a method of preparing a hydrogel, comprising a step of irradiating the above composition for forming hydrogel of the present invention with ultraviolet ray.

### EFFECT OF THE INVENTION

The composition according to the present invention forms a hydrogel in situ after being applied to tissues in the medical field and the hydrogel adheres to the tissues, and thus can be utilized as tissue adhesive, sealant, adhesion barrier, etc., and since properties (burst pressure, swelling ratio, degradation time, flexibility, etc.) thereof are sufficient to be utilized as sealant, it can play a role of sealing while maintaining the properties in the body during recovery of the patient’s wound area. In addition, since the gel formation time is as short as 10 seconds (more preferably, 5 seconds),

it is cured immediately after application to the tissue surface so as to make sealing of the wound area very easy, and it does not flow down surrounding organs so as to reduce unnecessary use of product, and due to the rapid gel formation, it can provide convenient use and reduced operation time. Furthermore, because of the flexibility and excellent adhesion, it can be used in moving organs such as lung, intestine, etc. Also, the composition and hydrogel according to the present invention can be employed in a medical device such as patch or implant material, etc., and can play a role of attaching the device to tissue, or a role of preventing adhesion between tissues after operation.

## BRIEF EXPLANATION OF THE DRAWINGS

Figure 1 is a scheme of synthesizing poly(γ-glutamic acid) having introduced methacrylic group as a photo-crosslinkable functional group (PGAGMA) by reacting glycidyl methacrylate (GMA) to the carboxyl group of poly(γ-glutamic acid) (PGA), according to an embodiment of the present invention.

Figure 2 is a scheme of preparing a hydrogel by crosslinking the poly(γ-glutamic acid) having introduced methacrylic group (PGAGMA) by ultraviolet ray (UV) irradiation in the presence of a photo-initiator, according to an embodiment of the present invention.

Figure 3 is a graph representing the results of measuring the swelling ratios of hydrogel according to substitution degrees of methacrylic group (MA), conducted in the Examples of the present invention.

Figure 4 is a graph representing the results of measuring the burst pressures of hydrogel according to substitution degrees of methacrylic group (MA), conducted in the Examples of the present invention.

Figure 5 represents photos showing flexibility of the hydrogel prepared in the Examples of the present invention.

## PREFERRED MODE FOR CARRYING OUT THE INVENTION

The present invention is explained in more detail below.

The composition for forming hydrogel of the present invention comprises a poly(γ-glutamic acid) (PGA) having a photo-crosslinkable functional group introduced therein; and a photo-initiator.

In an embodiment, the photo-crosslinkable functional group may be acrylate group, methacrylate group or a combination thereof.

In an embodiment, the photo-crosslinkable functional group may be derived from acrylate or methacrylate compound which can react with the carboxyl group of poly(γ-glutamic acid) and form a bond.

In an embodiment, such acrylate or methacrylate compound may be one or more selected from glycidyl methacrylate (GMA), 2-hydroxyethyl methacrylate, 2-aminoethyl methacrylate, glycidyl acrylate, 2-hydroxyethyl acrylate, 2-aminoethyl acrylate and salts thereof.

According to an embodiment, by the reaction of the above acrylate or methacrylate compound and poly(γ-glutamic acid), acrylate group or methacrylate group can be introduced as a photo-crosslinkable functional group in the poly(γ-glutamic acid).

In the present invention, "substitution degree of photo-crosslinkable functional group" means, based on 100% of the total moles of carboxyl group in the poly(γ-glutamic acid), the percent (%) of moles of the carboxyl group to which the photo-crosslinkable functional group is bonded.

According to an embodiment, the substitution degree of photo-crosslinkable functional group of the poly(γ-glutamic acid) comprised in the composition for forming hydrogel of the present invention is not especially limited, and for example, it may be 1% or more. If the substitution degree is too less than the above level, the properties of the hydrogel formed from the composition may deteriorate (decrease of burst pressure, increase of swelling ratio, etc.).

More concretely, the substitution degree of photo-crosslinkable functional group of the poly(γ-glutamic acid) may be, for example, 1% or more, 2% or more, 3% or more, 4% or more, 5% or more, 6% or more, 7% or more, 8% or more, 9% or more, 10% or more, 12% or more or 14% or more, and, it may be, for example, 30% or less, 28% or less, 26% or less, 24% or less, 22% or less or 20% or less. The upper limit of the substitution degree of photo-crosslinkable functional group of the poly(γ-glutamic acid) is not especially limited thereto, but if it exceeds the above level, the properties may not be improved further in spite of the increase of the substitution degree.

In an embodiment, the poly(γ-glutamic acid) comprised in the composition for forming hydrogel of the present invention may have a weight average molecular weight of from 10 kDa to 10000 kDa, and more concretely, from 100 kDa to 2000 kDa.

In an embodiment, the photo-initiator may form radical in UV A (320 to 400 nm) region, and preferably it may be a photo-initiator having the maximum absorbance in UV A region. Since the light in UV B (280 to 320 nm) and UV C (100 to 180 nm) regions has a short wavelength and so a high energy, it may cause damage to the tissue and thus application thereof to a living body is inappropriate. Accordingly, in case of irradiation with light in UV A region, use of

a photo-initiator having the maximum absorbance in UV B and UV C regions is inappropriate due to its poor radical generation efficiency.

According to an embodiment of the present invention, as a photo-initiator having the maximum absorbance in UV A region, the photo-initiator may be phosphorus-containing aromatic photo-initiator compound, azo-based photo-initiator compound, flavin-based photo-initiator compound, or a combination thereof, but it is not limited thereto.

In an embodiment, the phosphorus-containing aromatic photo-initiator compound may be aromatic phosphinate, aromatic phosphonate, aromatic phosphine oxide, or a combination thereof, and, for example, as that having aromatic group such as benzoyl or terephthaloyl, it may be phosphinate or a salt thereof, phosphonate or a salt thereof, phosphine oxide or a salt thereof, or a combination thereof, but it is not limited thereto.

More concretely, the phosphorus-containing aromatic photo-initiator compound may be lithium phenyl-2,4,6-trimethylbenzoylphosphinate (LAP) (maximum absorbance wavelength: 375 nm) of the following structure, lithium bisacylphosphine oxide (BAPO-OLi) (e.g., lithium phenylbis(2,4,6-trimethylbenzoyl)phosphine oxide) (maximum absorbance wavelength: 375 nm), or a combination thereof, and still more concretely, it may be LAP.

[LAP]

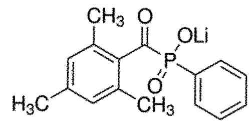

In an embodiment, the azo-based photo-initiator compound may be azo nitrile-based, azo ester-based, azo amide-based, azo amidine-based, or macroazo-based photo-initiator compound, or a combination thereof, but it is not limited thereto.

More concretely, the azo nitrile-based photo-initiator may be V-60, V-65, V-59, V-70, V-40, etc. of Wako Pure chemical Industris. Ltd., the azo ester-based photo-initiator may be V-601, etc. of Wako Pure chemical Industris. Ltd., the azo amide-based photo-initiator may be VA-086, VA-085, VA-080, Vam-110, Vam-111, VF-096, etc. of Wako Pure chemical Industris. Ltd., the azo amidine-based photo-initiator may be V-50, VA-044, VA-046B, Aam-027, VA060, VA-057, VA-061, etc. of Wako Pure chemical Industris. Ltd., and the macroazo-based photo-initiator may be VPS-0501, VPS-1001, VPE-0201, VPE-0401, VPE-0601, VPTG-0301, etc. of Wako Pure chemical Industris. Ltd., but it is not limited thereto.

Still more concretely, the azo-based photo-initiator compound may be an azo amide-based photo-initiator compound, for example, 2,2'-Azobis[2-methyl-N-(2-hydroxyethyl)propionamide] (VA-086) (maximum absorbance wavelength: 365 nm).

In an embodiment, the flavin-based photo-initiator compound may be riboflavin, but it is not limited thereto.

In an embodiment, the concentration of the photo-initiator comprised in the composition for forming hydrogel of the present invention is not especially limited, and it may be a concentration suitable for forming hydrogel by irradiation. For instance, the concentration of the photo-initiator comprised in the composition may be 0.1 mM or higher, 0.5 mM or higher, 1 mM or higher or 1.5 mM or higher, and it maybe 10 mM or lower, 7 mM or lower, 5 mM or lower, or 3 mM or lower, but it is not limited thereto.

In an embodiment, when irradiated with ultraviolet ray, preferably irradiated with ultraviolet ray in UV A region (for example, 365 nm wavelength), the composition for forming hydrogel of the present invention may be crosslinked within 10 seconds (more preferably, within 5 seconds) (for example, 1 to 10 seconds, or 1 to 5 seconds) and may form hydrogel. At this time, the wavelength of the ultraviolet ray inducing the photo-crosslinking may be selected properly according to the type of photo-initiator comprised in the composition. For instance, when lithium phenyl-2,4,6-trimethylbenzoylphosphinate (LAP) is used as the photo-initiator, the wavelength of the ultraviolet ray inducing the photo-crosslinking may be, for example, 365 nm.

Thus, other aspects of the present invention provide a hydrogel formed by photo-crosslinking the composition for forming hydrogel of the present invention, and a method of preparing a hydrogel, comprising a step of irradiating the composition for forming hydrogel of the present invention with ultraviolet ray, and more preferably, with ultraviolet ray in UV A region.

In an embodiment, the composition for forming hydrogel of the present invention may be a composition for forming hydrogel, comprising: a first component comprising a poly($\gamma$-glutamic acid) (PGA) having a photo-crosslinkable functional group introduced therein; and a second component comprising a photo-initiator.

Thus, when applied to the tissue surface and then irradiated with ultraviolet ray, the composition for forming hydrogel of the present invention is crosslinked in situ, preferably within 10 seconds (more preferably, within 5 seconds) and forms hydrogel, and the hydrogel can perform tissue adhesion or sealing.

Thus, according to an embodiment, the hydrogel of the present invention can be utilized for use of tissue adhesive, sealant, or adhesion barrier.

The present invention is explained in more detail through the following Examples. However, the following examples are only to illustrate the present invention, and the scope of the present invention is not limited thereby in any manner.

**[EXAMPLES]**

**Preparation of poly(γ-glutamic acid) having a photo-crosslinkable functional group introduced therein**

Poly(γ-glutamic acid) (PGA) and glycidyl methacrylate (GMA) were reacted to prepare poly(γ-glutamic acid) having introduced methacrylic (MA) group as a photo-crosslinkable functional group (PGAGMA). The substitution degree of MA in the prepared PGAGMA was controlled by changing the amount of GMA reacting with PGA.

**Preparation of the composition for forming hydrogel and the hydrogel**

The above-prepared poly(γ-glutamic acid) having introduced methacrylic (MA) group (PGAGMA) and a photo-initiator were dissolved in a buffer to prepare the composition for forming hydrogel.

As the photo-initiator of Examples, lithium phenyl-2,4,6-trimethylbenzoylphosphinate (LAP) and 2,2'-azobis[2-methyl-N-(2-hydroxyethyl)propionamide] (VA-086) were used, whereas as the photo-initiator of Comparative Examples, Irgacure 2959 of the following structure was used.

[Irgacure 2959]

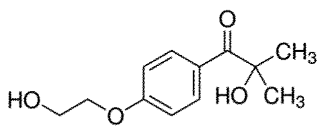

The prepared composition for forming hydrogel was irradiated with ultraviolet ray (UV) of 365 nm wavelength for photo-crosslinking, and thereby the hydrogel was formed.

**Experimental Example**

**(1) Measurement of gelation time**

For each of the compositions prepared with various types and concentrations of photo-initiator as shown in Table 1 below, the gelation time was measured, and the results are shown in Table 1 below.

[Table 1]

| Polymer | Type of photo-initiator | Concentration of photo-initiator (mM) | UV intensity ($mW/cm^2$) | Gelation time (second) |
|---|---|---|---|---|
| PGAGMA | LAP | 2 | 15 | 2.3±0.1 |
| | | | 30 | 1.8±0.1 |
| | | | 100 | 1.3±0.1 |
| | VA-086 | 2 | 30 | 43.7±7.3 |
| | | | 100 | 21.0±1.4 |
| | | | 150 | 16.3±0.9 |
| | | 10 | 30 | 16.0±1.2 |
| | | | 100 | 7.8±0.4 |
| | Irgacure 2959 | 2 | 150 | No gelation |
| | | 10 | 15 | 185.7±42.4 |
| | | | 30 | 80.3±2.9 |
| | | 25 | 30 | 41.0±6.5 |
| | | | 100 | 26.0±0.0 |
| | | | 150 | 20.7±0.5 |
| - PGAGMA: poly(y-glutamic acid) having introduced methacrylic (MA) group<br>- LAP: lithium phenyl-2,4,6-trimethylbenzoylphosphinate<br>- VA-086: 2,2'-azobis[2-methyl-N-(2-hydroxyethyl)propionamide] | | | | |

As shown in Table 1 above, in case of using LAP and VA-086 as the photo-initiator, the radical generation efficiency was high when irradiated with ultraviolet ray of 365 nm wavelength, i.e., in UV A region (320 to 400 nm), and thus gel formation was possible within short time even with relatively low photo-initiator concentration and low light intensity (UV intensity).

To the contrary, in case of using Irgacure 2959 as the photo-initiator, gel was not formed when the photo-initiator concentration was low, and it was hard to shorten the gelation time within 10 seconds even if the light intensity was increased.

When a gelation time is too long (i.e., 10 seconds or longer), the composition flows down before gelation so that the gel is difficult to apply and adhere to the desired area, and thus it is not suitable for use as a sealant.

**(2) Measurement of swelling ratio of hydrogel according to substitution degree of MA**

Each of the compositions comprising PGAGMA prepared with changing **substitution degree of MA** and LAP (2mM) was irradiated with ultraviolet ray of 365 nm wavelength to form hydrogel.

Then, the initial weight (W0) of the prepared hydrogel was measured and the sample was immersed in PBS, and then stored in a shaking incubator at 37°C for 24 hours. Then, the excess water on the sample surface was sufficiently wiped off, the weight of the sample (W1) was measured, and the swelling ratio was calculated through the following equation, and the results are shown in Figure 3.

$$\text{Swelling ratio (\%)} = ((W1-W0)/W0)*100\%$$

As shown in Figure 3, in case where the **substitution degree of MA** of PGAGMA was 6% or higher, the hydrogel showed a tendency of low swelling ratio as 50% or less (tendency of less absorption of water), whereas in case where the **substitution degree of MA** of PGAGMA was less than 6%, the hydrogel showed a tendency of very high swelling ratio as 350% level. Thus, it can be confirmed that the swelling ratio has a tendency of increase as the substitution degree decreases.

### (3) Measurement of burst pressure of hydrogel according to substitution degree of MA

Each of the compositions comprising PGAGMA prepared with changing MA substitution degree and LAP (2mM) was irradiated with ultraviolet ray of 365 nm wavelength to form hydrogel.

The following test was conducted according to ASTM2392-04 (Standard Test Method for Burst Strength of Surgical Sealants), and the results are shown in Figure 4.

The collagen casing was washed sequentially with distilled water and ethanol, and the washed collagen casing was punched to make a 3 mm hole therein, and then a teflon ring (inner diameter: 15 mm, thickness: 2 mm) was placed on the center of the hole.

Then, each of the compositions comprising PGAGMA prepared with changing **substitution degree of MA** and LAP (2mM) was irradiated with ultraviolet ray of 365 nm wavelength to form hydrogel.

Then, the hydrogel-attached collagen casing was mounted on an instrument for measuring burst pressure, and whiled flowing PBS at a rate of 2 mL/min, applied maximum hydraulic pressure was measured.

As shown in Figure 4, in case where the **substitution degree of MA** of PGAGMA was 6% or higher, the burst pressure of the hydrogel was very high as 300 mmHg or higher, whereas in case where the MA substitution degree was less than 6%, the burst pressure of the hydrogel was low as 100 mmHg level. Thus, it can be confirmed that the burst pressure degreases as the substitution degree decreases.

### (4) Flexibility of hydrogel

While bending the hydrogel sample prepared according to the present invention by hand, photos thereof were taken as shown in Figure 5.

As shown in Figure 5, the hydrogel of the present invention showed good flexibility, and thus it can be adhered to moving organs while maintaining its shape, and so it can be utilized for use of tissue adhesive, sealant, or agent for preventing adhesion between tissues applied to lung, intestine, etc.

## Claims

**1.** A composition for forming hydrogel, comprising:

a poly(γ-glutamic acid) (PGA) having a photo-crosslinkable functional group introduced therein; and
a photo-initiator.

**2.** The composition for forming hydrogel of claim 1, wherein the photo-crosslinkable functional group is acrylate group, methacrylate group or a combination thereof.

**3.** The composition for forming hydrogel of claim 2, wherein the photo-crosslinkable functional group is derived from acrylate or methacrylate compound which can react with the carboxyl group of poly(γ-glutamic acid) and form a bond.

**4.** The composition for forming hydrogel of claim 3, wherein the acrylate or methacrylate compound is one or more selected from glycidyl methacrylate (GMA), 2-hydroxyethyl methacrylate, 2-aminoethyl methacrylate, glycidyl acrylate, 2-hydroxyethyl acrylate, 2-aminoethyl acrylate and salts thereof.

**5.** The composition for forming hydrogel of claim 1, wherein the photo-initiator is a photo-initiator having the maximum absorbance in UV A region.

**6.** The composition for forming hydrogel of claim 5, wherein the photo-initiator is phosphorus-containing aromatic photo-initiator compound, azo-based photo-initiator compound, flavin-based photo-initiator compound, or a combination thereof.

**7.** The composition for forming hydrogel of claim 1, which is crosslinked within 10 seconds and forms hydrogel when irradiated with ultraviolet ray.

**8.** A hydrogel formed by photo-crosslinking a composition for forming hydrogel of any one of Claims 1 to 7.

**9.** The hydrogel of claim 8, which is for use of tissue adhesive, sealant, or adhesion barrier.

**10.** A method of preparing a hydrogel, comprising a step of irradiating a composition for forming hydrogel of any one of Claims 1 to 7 with ultraviolet ray in UV A region.

【FIGURE 1】

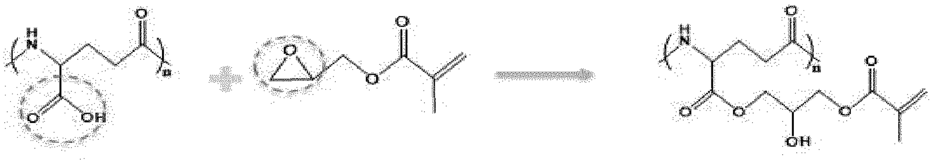

PGA GMA PGAGMA

【FIGURE 2】

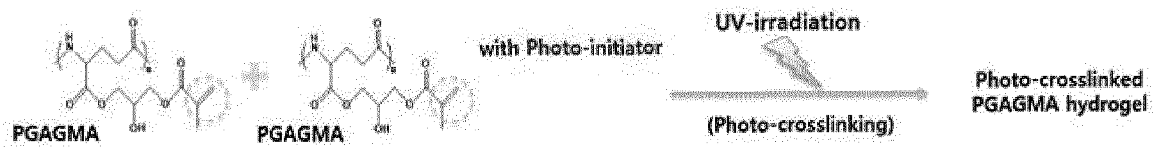


【FIGURE 3】

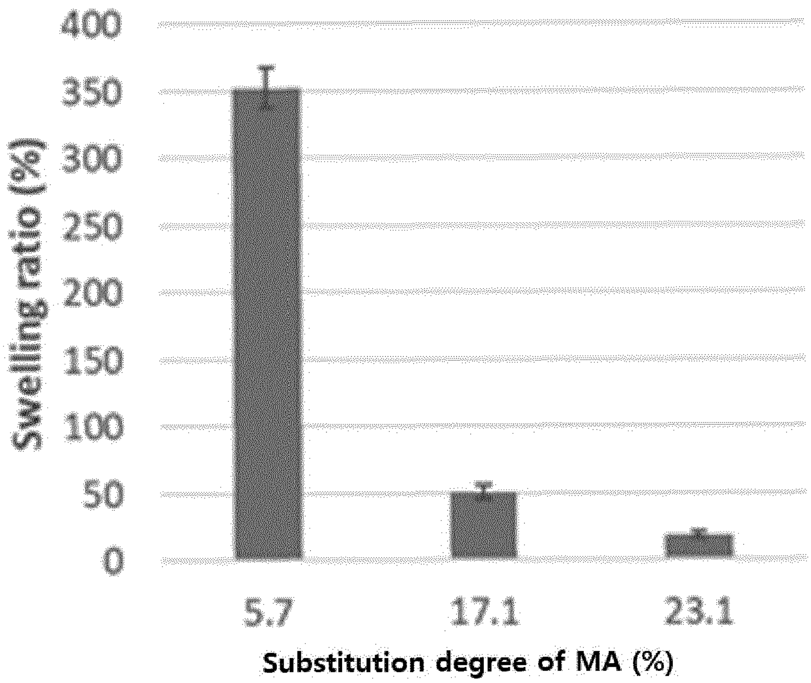

【FIGURE 4】

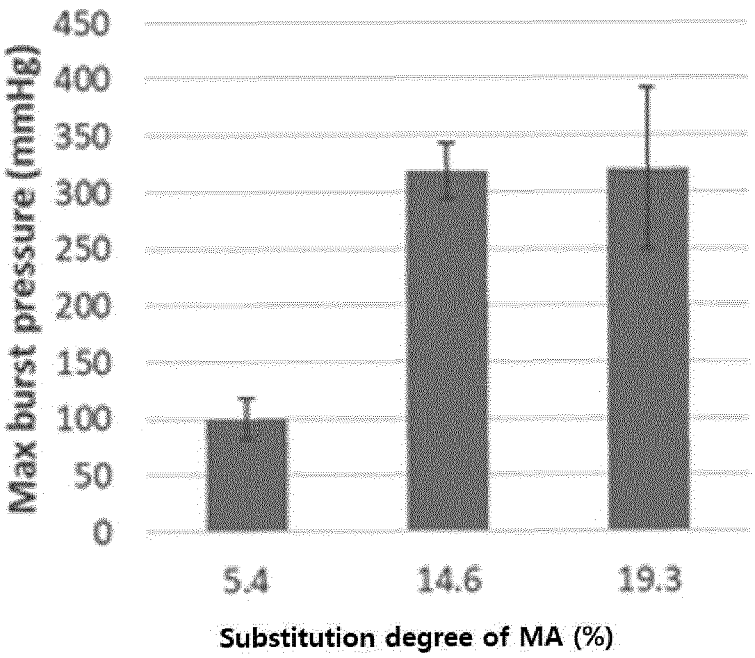


【FIGURE 5】

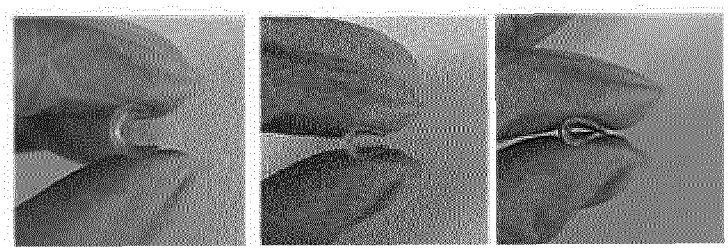

# INTERNATIONAL SEARCH REPORT

International application No.
**PCT/KR2021/010330**

## A. CLASSIFICATION OF SUBJECT MATTER

**C08L 77/04**(2006.01)i; **C08G 63/08**(2006.01)i; **C08G 63/91**(2006.01)i; **C08J 3/28**(2006.01)i; **C08J 3/24**(2006.01)i; **A61L 24/00**(2006.01)i; **A61L 24/06**(2006.01)i; **A61L 31/14**(2006.01)i; **A61L 31/04**(2006.01)i

According to International Patent Classification (IPC) or to both national classification and IPC

## B. FIELDS SEARCHED

Minimum documentation searched (classification system followed by classification symbols)

C08L 77/04(2006.01); A61L 27/18(2006.01); A61L 27/50(2006.01); C07D 291/08(2006.01); C07K 2/00(2006.01); C08G 69/10(2006.01); C08G 69/48(2006.01); C08J 3/075(2006.01); C08J 3/28(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 수화겔(hydrogel), 폴리(감마-글루탐산)(poly(gamma-glutamic acid)), 광개시제(photo initiator), 아크릴레이트(acrylate), 메타크릴레이트(methacrylate)

## C. DOCUMENTS CONSIDERED TO BE RELEVANT

| Category* | Citation of document, with indication, where appropriate, of the relevant passages | Relevant to claim No. |
|---|---|---|
| X<br>Y | CN 111253592 A (NANJING TECH UNIVERSITY) 09 June 2020 (2020-06-09)<br>See claims 1-9; and paragraphs [0024]-[0032], [0036]-[0038] and [0065]. | 1-4,8-10<br>5-7 |
| Y | CN 109776451 A (ZHONGSHAN GUANGHE MEDICAL TECHNOLOGY CO., LTD.) 21 May 2019 (2019-05-21)<br>See paragraphs [0203], [0204] and [0941]; and table 2. | 5-7 |
| A | CN 109045354 A (HUAZHONG UNIVERSITY OF SCIENCE AND TECHNOLOGY) 21 December 2018 (2018-12-21)<br>See entire document. | 1-10 |
| A | KR 10-2020-0001456 A (KOOKMIN UNIVERSITY INDUSTRY ACADEMY COOPERATION FOUNDATION) 06 January 2020 (2020-01-06)<br>See entire document. | 1-10 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **23 November 2021** | **23 November 2021** |
| Name and mailing address of the ISA/KR<br>**Korean Intellectual Property Office**<br>**Government Complex-Daejeon Building 4, 189 Cheongsa-ro, Seo-gu, Daejeon 35208**<br>Facsimile No. **+82-42-481-8578** | Authorized officer<br><br>Telephone No. |

Form PCT/ISA/210 (second sheet) (July 2019)

INTERNATIONAL SEARCH REPORT

International application No.
**PCT/KR2021/010330**

**C. DOCUMENTS CONSIDERED TO BE RELEVANT**

| Category* | Citation of document, with indication, where appropriate, of the relevant passages | Relevant to claim No. |
|---|---|---|
| A | KR 10-2013-0078549 A (SAMYANG BIOPHARMACEUTICALS CORPORATION) 10 July 2013 (2013-07-10)<br>See entire document. | 1-10 |

Form PCT/ISA/210 (second sheet) (July 2019)

**INTERNATIONAL SEARCH REPORT**
**Information on patent family members**

International application No.
**PCT/KR2021/010330**

| Patent document cited in search report | Publication date (day/month/year) | Patent family member(s) | Publication date (day/month/year) |
|---|---|---|---|
| CN 111253592 A | 09 June 2020 | None | |
| CN 109776451 A | 21 May 2019 | CN 109776451 B | 11 December 2020 |
| | | CN 112266367 A | 26 January 2021 |
| | | CN 112266368 A | 26 January 2021 |
| | | EP 3666761 A1 | 17 June 2020 |
| | | EP 3666761 A4 | 30 December 2020 |
| | | JP 2020-533287 A | 19 November 2020 |
| | | US 1117879 B2 | 14 September 2021 |
| | | US 2020-0262802 A1 | 20 August 2020 |
| | | WO 2019-095599 A1 | 23 May 2019 |
| CN 109045354 A | 21 December 2018 | CN 109045354 B | 08 December 2020 |
| KR 10-2020-0001456 A | 06 January 2020 | CN 112334518 A | 05 February 2021 |
| | | JP 2021-522027 A | 30 August 2021 |
| | | JP 6945088 B2 | 06 October 2021 |
| | | KR 10-2163563 B1 | 08 October 2020 |
| | | US 1111340 B2 | 07 September 2021 |
| | | US 2021-0139655 A1 | 13 May 2021 |
| | | WO 2020-004810 A1 | 02 January 2020 |
| KR 10-2013-0078549 A | 10 July 2013 | EP 2797984 A1 | 05 November 2014 |
| | | EP 2797984 A4 | 12 August 2015 |
| | | EP 2797984 B1 | 07 December 2016 |
| | | ES 2610302 T3 | 26 April 2017 |
| | | KR 10-1444877 B1 | 01 October 2014 |
| | | US 2014-0336276 A1 | 13 November 2014 |
| | | US 9254348 B2 | 09 February 2016 |
| | | WO 2013-100715 A1 | 04 July 2013 |

Form PCT/ISA/210 (patent family annex) (July 2019)